# EUROPÄISCHE PATENTANMELDUNG

(11) **EP 3 839 540 A1**
(43) Veröffentlichungstag der Anmeldung: **23.06.2021**
(21) Anmeldenummer: 19218683.1
(22) Anmeldetag: 20.12.2019
(51) Int. Cl.: G01R 33/28, G01R 33/48

(54) **BETREIBEN EINES MR-SYSTEMS SOWIE MR-SYSTEM**

(71) Anmelder: Siemens Healthcare GmbH, 91052 Erlangen (DE)
(72) Erfinder: Biber, Stephan, 91056 Erlangen (DE)

(57) **Zusammenfassung**

Ein Verfahren dient zum Betreiben eines MR-Systems (1, 2, 3) mit mindestens einer MR-Körperspule (1) und einer mit der mindestens einen MR-Körperspule (1) verbundenen Steuereinrichtung (3), wobei mittels mindestens eines Radiometers (6) eine Körpertemperatur eines durch das jeweilige Radiometer (6) ausleuchtbaren Körperbereichs eines durch das MR-System (1, 2, 3) zu untersuchenden Patienten (P) gemessen wird, die gemessene Körpertemperatur mit einer Grenztemperatur verglichen wird und eine auf den Patienten eingestrahlte MR-Sendeleistung beruhend auf einem Ergebnis des Vergleichs an die Grenztemperatur angenähert wird. Das Radiometer (6) arbeitet insbesondere in einem von dem MR-Band unterschiedlichen Frequenzband. Eine MR-Körperspule (1) zur Durchführung des Verfahrens weist mindestens eine Radiometerantenne (7) und einen der Radiometerantenne (7) nachgeschalteten Verstärker (11) auf. Die MR-Körperspule (1) kann ferner ein der Radiometerantenne (7) nachgeschaltetes Eingangs-Sperrfilter (10), das für das MR-Band sperrend ausgelegt ist, aufweisen.

## Beschreibung

Die Erfindung betrifft ein Verfahren zum Betreiben eines MR-Systems mit mindestens einer MR-Körperspule und einer mit der mindestens einen MR-Körperspule verbundenen Steuereinrichtung, bei dem mittels mindestens eines Radiometers die Körpertemperatur eines durch das jeweilige Radiometer ausleuchtbaren Körperbereichs eines durch das MR-System zu untersuchenden Patienten gemessen wird. Die Erfindung betrifft auch eine zur Durchführung des Verfahrens eingerichtete MR-Körperspule und ein zur Durchführung des Verfahrens eingerichtetes MR-System.

In der Kernspintomographie (MRT) werden MR-Sendesysteme zum Anregen der Spins im Körper eines Patienten benutzt. Dazu werden mittlere Leistungen im Bereich von einigen 100 W abgegeben, wobei auch sehr hohe Spitzenleistungen im Bereich von 1 kW bis 40 kW benötigt werden. Ein Problem besteht dabei darin, dass insbesondere bei Hochfeldsystemen mit einer Stärke eines B0-Felds von mehr als einem Tesla so hohe mittlere Leistungen (SAR Spezifische Absorptionsrate W/kg) im Patienten absorbiert werden, dass diese überwacht werden müssen, um zulässige SAR-Grenzwerte nicht zu überschreiten. Zur Bestimmung der SAR-Grenzwerte werden häufig MR-Körpermodelle hinterlegt und eine Lage des Patienten abgeschätzt. Beruhend auf diesen Informationen, den Anforderungen der MR-Sequenz(en) eines MR-Scans und von Reflexionsfaktoren der MR-Sendespule(n) kann abgeschätzt werden, wieviel mittlere MR-Sendeleistung abgegeben werden kann, ohne die SAR-Grenzwerte zu überschreiten. Damit unter allen Bedingungen die Sicherheit des Patienten gewährleistet werden kann, werden hohe Sicherheitsfaktoren eingerechnet, die nachteiligerweise zu stärkeren Einschränkungen der MR-Sendeleistung führen, als in vielen Fällen nötig ist.

AbdEl-Monem M. El-Sharkawy et al., "Absolute Temperature Monitoring Using RF Radiometry in the MRI Scanner", IEEE Trans Circuits Syst I Regul Pap. Author manuscript; available in PMC 2007 April 10, Published in final edited form as: IEEE Trans Circuits Syst I Regul Pap. 2006 November; 53(11): 2396-2404, betrifft eine Temperaturerfassung mittels Mikrowellenradiometrie zur nichtinvasiven Messung einer absoluten Temperatur von Geweben in einem Körper. Derzeitige klinische Radiometer arbeiten jedoch im Gigahertz-Bereich, was ihre Eindringtiefe begrenzt. Deshalb wird ein nicht-invasives Radiometer vorgestellt, das bei niedrigen Radiofrequenzen (64 MHz) mit einer Bandbreite von 100 kHz mit einer externen HF-Schleifenspule als thermischem Detektor arbeitet. Das Radiometer nutzt eine genaue Impedanzmessung und eine automatische Anpassungsschaltung mit einer Genauigkeit von 0,05 Ω, um eventuelle Lastschwankungen auszugleichen. Das Radiometer ermöglicht Temperaturmessungen mit einer Genauigkeit von ± 0,1 °K über einen getesteten physiologischen Bereich von 28 °C bis 40 °C in Salzphantomen, deren elektrische Eigenschaften denen von Gewebe entsprechen. Da 1,5-T-Magnetresonanztomographen (MRT) auch bei 64 MHz arbeiten, wird gezeigt, dass die Integration eines Radiometers in einen MRT-Scanner zur Überwachung einer HF-Leistungsabgabe und der Temperaturdosimetrie möglich ist, um grobe, ortsaufgelöste, absolute Wärmekarten im physiologischen Bereich zu erhalten . Es wird daraus geschlossen, dass die HF-Radiometrie als direkte, nicht-invasive Methode zur Überwachung einer Gewebeerwärmung während MRT-Messungen vielversprechend ist und damit ein unabhängiges Mittel zur Überprüfung der Patientensicherheit darstellt.

Es ist die **Aufgabe** der vorliegenden Erfindung, die Nachteile des Standes der Technik zumindest teilweise zu überwinden und insbesondere eine verbesserte Möglichkeit zur Anpassung einer MR-TX-Leistung während einer MR-Untersuchung an einem Patienten bereitzustellen.

Diese Aufgabe wird gemäß den Merkmalen der unabhängigen Ansprüche gelöst. Bevorzugte Ausführungsformen sind insbesondere den abhängigen Ansprüchen entnehmbar.

Die Aufgabe wird gelöst durch ein Verfahren zum Betreiben eines MR-Systems mit mindestens einer MR-Körperspule und einer mit der mindestens einen MR-Körperspule verbundenen Steuereinrichtung, bei dem
- mittels mindestens eines Radiometers die Körpertemperatur eines durch das jeweilige Radiometer ausleuchtbaren Körperbereichs eines durch das MR-System zu untersuchenden Patienten gemessen wird,
- die gemessene Körpertemperatur mit einer Grenztemperatur verglichen wird und
- eine auf den Patienten eingestrahlte MR-Sendeleistung beruhend auf einem Ergebnis des Vergleichs an die Grenztemperatur angenähert wird, insbesondere aber nicht überschritten wird. Mit anderen Worten wird eine auf den Patienten eingestrahlte MR-Sendeleistung beruhend auf einem Ergebnis des Vergleichs so eingestellt, dass die, insbesondere mittels des mindestens einen Radiometers messbare, Körpertemperatur an die Grenztemperatur angenähert wird, insbesondere aber nicht überschritten wird.

Dieses Verfahren ergibt den Vorteil, dass während eines MR-Messvorgangs (auch als "MR-Scan" bezeichenbar) mindestens eine Körpertemperatur schnell und nicht-invasiv bestimmbar ist. Dies wiederum kann dazu verwendet werden, die auf den Patienten eingestrahlte MR-Sendeleistung (auch als "MR-TX-Leistung" bezeichenbar) an vorgeschriebene Grenz-Körpertemperaturen anzupassen. Dadurch kann sichergestellt werden, dass eine erlaubte MR-Sendeleistung ausgeschöpft wird, so dass sich eine Bildqualität und/oder Messgeschwindigkeit erhöhen lässt. Ein weiterer Vorteil besteht darin, dass sich die Körpertemperatur, beispielsweise im Gegensatz zu einer IR-Bildgebung, auch im Inneren des Patienten feststellen lässt, auch im Inneren des Patienten messen lässt, was eine noch bessere Annäherung an Grenz-Körpertemperaturen erlaubt. Dabei sind auch räumliche (spatiale) Temperaturverteilungen in dem Patienten messbar, so dass die MR-Sendeleistung auch in Abhängigkeit von lokalen Temperaturen anpassbar ist, was eine noch sicherere Einhaltung von Grenzwerten ermöglicht und/oder eine noch bessere Ausnutzung der MR-Sendeleistung ermöglicht.

Das MR-System umfasst mindestens eine MR-Körperspule und eine mit der mindestens einen MR-Körperspule verbundene Steuereinrichtung. Die MR-Körperspule umfasst mindestens eine oder mehrere MR-Empfangsantennen, welche eine Signalantwort des Patienten auf in ihn eingestrahlte MR-Signale (auch als "MR-TX-Signale" bezeichenbar, z.B. MR-Pulse) empfangen und an das MR-System übermitteln. Die MR-TX-Signale können beispielsweise von einem MR-Gerät (auch als "MR-Scanner" bezeichenbar) und/oder von an der Körperspule vorhandenen MR-Sendeantennen erzeugt werden. Im letzteren Fall können die MR-Sendeantennen den MR-Empfangsantennen entsprechen oder unterschiedliche Antennen sein. Ein solches MR-System (ohne Berücksichtigung der radiometrischen Temperaturmessung) ist grundsätzlich bekannt und wird deshalb nicht mehr weiter ausgeführt.

Unter einem durch ein Radiometer ausleuchtbaren Körperbereich wird insbesondere ein Körperbereich verstanden, dessen Temperatur durch dieses Radiometer bestimmbar oder messbar ist. Dabei kann die Körpertemperatur für diesen Körperbereich insbesondere als ein Wert bestimmt werden. Eine Temperaturverteilung des Patienten lässt sich z.B. durch eine Anordnung mehrerer Radiometer mit unterschiedlichen ausleuchtbaren Körperbereichen messen, was auch als "Spatial Diversity" bezeichnet werden kann. Das Prinzip einer radiometrischen Messung einer Körpertemperatur wird als grundsätzlich bekannt vorausgesetzt.

Es ist eine Weiterbildung, dass mittels des mindestens einen Radiometers die Körpertemperatur des zu untersuchenden Patienten in verschiedenen Tiefen des Patienten gemessen wird. So lässt sich vorteilhafterweise die MR-Sendeleistung auch in Abhängigkeit von lokal unterschiedlich tiefen Temperaturen anpassen, was eine noch sicherere Einhaltung von Grenzwerten ermöglicht und/oder eine noch bessere Ausnutzung der MR-Sendeleistung ermöglicht. Die Messung der Körpertemperatur in unterschiedlichen Tiefen lässt sich beispielsweise so umsetzen, dass das mittels des mindestens einen Radiometers aufgenommene Radiometersignal in verschiedenen Frequenzen und/oder Frequenzbereichen ausgewertet wird (z.B. bei 500 MHz, 1 GHz, 2 GHz, 5 GHz und/oder 10 Ghz), da durch unterschiedliche Frequenzen unterschiedlich tief im Körper liegende Anatomien betrachtet werden können. Dies kann auch als "Frequency Diversity" bezeichnet werden. Besonders vorteilhaft für eine Bestimmung lokaler Körpertemperaturen in drei Dimensionen (z.B. in der durch die mehreren Radiometer aufgezogenen Ebene und in der Tiefe) ist die Anordnung von mehreren Radiometern an unterschiedlichen Raumpositionen mit unterschiedlichen Ausleuchtbereichen, deren Radiometersignale in verschiedenen Frequenzen und/oder Frequenzbereichen ausgewertet werden.

Die zur radiometrischen Temperaturmessung typischerweise eingesetzte Radiometerantenne ist insbesondere eine von einer MR-Antenne unterschiedliche Antenne. Dadurch wird der Vorteil erreicht, dass eine Beeinflussung der radiometrischen Temperaturmessung durch MR-Signale gering gehalten werden kann. Auch kann so eine Bandbreite des für die radiometrischen Temperaturmessung nutzbaren Frequenzbands oder Nutzbands besonders breit gehalten werden, während MR-Antennen nachteiligerweise eher schmalbandig auf die MR-Frequenzen ausgelegt sind.

Die Grenztemperatur ist insbesondere eine vorgegebene maximale Grenztemperatur. Sie kann z.B. vorgegeben sein, um eine Schädigung des Patienten zu verhindern. Es ist eine Weiterbildung, dass für einen Patienten genau eine Grenztemperatur vorgegeben ist. Es ist eine Weiterbildung, dass für unterschiedliche Körperbereiche individuelle, ggf. auch unterschiedliche, Grenztemperaturen vorgegeben sind.

Die Grenztemperatur(en) kann bzw. können z.B. aufgrund körperphysiologische Betrachtungen erlangt worden sein. Beispielsweise kann bzw. können die Grenztemperatur(en) aus der SAR ("Spezifische Absorptionsrate") abgeleitet werden, die ein Patient während eines MR-Scans nicht überschreiten sollte. Dass die gemessene Körpertemperatur mit einer Grenztemperatur verglichen wird, kann aber auch umfassen, dass die gemessene Körpertemperatur in SAR-Werte umgerechnet wird und diese umgerechneten SAR-Werte mit SAR-Grenzwerten verglichen werden.

Dass eine auf den Patienten eingestrahlte MR-Sendeleistung beruhend auf einem Ergebnis des Vergleichs an die Grenztemperatur angenähert wird, aber nicht überschritten wird, kann beispielsweise bedeuten, dass
- dann, wenn die gemessene Körpertemperatur unterhalb der, ggf. lokalen, Grenztemperatur liegt, die MR-Sendeleistung erhöht wird, bis die Grenztemperatur erreicht oder gerade noch nicht erreicht ist;
- dann, wenn die gemessene Körpertemperatur oberhalb der, ggf. lokalen, Grenztemperatur liegt, die MR-Sendeleistung erniedrigt wird, bis die Grenztemperatur wieder erreicht oder etwas geringer ist.

Das Verfahren kann automatisch durch das MR-Gerät durchgeführt werden. Alternativ oder zusätzlich kann das MR-Gerät, insbesondere dessen Steuereinrichtung, dazu eingerichtet sein, die MR-Sendeleistung nach entsprechender Eingabe durch einen Nutzer einzustellen.

Das Verfahren kann ein oder mehrere Radiometer nutzen. Mehrere Radiometer können insbesondere dazu genutzt werden, die Temperatur an unterschiedlichen Körperbereichen zu messen und dadurch eine Temperaturverteilung eines Patienten zu messen. Dass die gemessene Körpertemperatur mit einer Grenztemperatur verglichen wird kann dann beispielsweise folgende Fälle umfassen:
- durch unterschiedliche Radiometer gemessene Körpertemperaturen werden mit einer für alle Körperbereiche gleichen Grenztemperatur verglichen;
- durch unterschiedliche Radiometer gemessene Körpertemperaturen werden mit einer für die Körperbereiche individuellen Grenztemperaturen verglichen.

Es ist eine Ausgestaltung, dass das Radiometer ein Dicke-Radiometer ist, das eine Antenne ("Radiometerantenne"), eine Rauschquelle und einen Dicke-Schalter aufweist und der Dicke-Schalter abwechselnd mit einer bestimmten Frequenz zwischen der Radiometerantenne und der Rauschquelle umgeschaltet wird. Ein Dicke-Radiometer ermöglicht vorteilhafterweise einen besonders einfachen Aufbau. Durch das Umschalten werden thermische Drifteffekte, die deutlich länger als die Umschaltzeit des Dicke-Schalters sind, eliminiert (Dicke-Prinzip), wobei eine typische Frequenz für die Umschaltung im Bereich zwischen 5 Hz und 50 kHz liegt.

Das von der Radiometerantenne aufgenommene Messignal ist ein Rauschsignal, das einem thermischen (Planck'schen) Rauschen des Patienten entspricht. Die Rauschquelle (die auch als "Rauschgenerator" oder "Referenz-Rauschquelle" bezeichenbar ist) erzeugt ein Referenz-Rauschsignal. Unter einem Rauschsignal kann daher im Folgenden, wenn sich aus dem Zusammenhang nichts anderes ergibt, sowohl das von der Rauschquelle künstlich erzeugte Referenz-Rauschsignal als auch das von der Radiometerantenne aufgenommene Messsignal verstanden werden. Die Rauschquelle kann beispielsweise ein 50 Ohm-Widerstand oder eine kalibrierte Rauschquelle, beispielsweise auf Basis einer Rauschdiode, sein.

Durch Vergleich der beiden Rauschleistungen von Radiometerantenne (Messsignal) und Rauschquelle (Referenz-Rauschsignal) kann die Rauschleistung des Messsignals bestimmt werden. Aus der Kenntnis der Rauschleistung P des von der Radiometerantenne empfangenen Messsignals, der Bandbreite B des Nutzbands und des Kettengewinns G der Empfangskette kann die durch die Radiometerantenne gemessene Temperatur T gemäß T = P / (k . B . G) mit k der Boltzmann-Konstanten absolut bestimmt werden. Der Aufbau und die Arbeitsweise von Dicke-Radiometern sind grundsätzlich bekannt, so dass hierzu nicht weiter ausgeführt wird.

Es ist eine Ausgestaltung, dass der Dicke-Schalter während Sendephasen des MR-Systems mit der Rauschquelle verbunden wird oder ist und während Nichtsendephasen des MR-Systems mit der Radiometerantenne verbunden wird oder ist. So wird der Vorteil erreicht, dass eine Beeinflussung der Temperaturnmessung über die Radiometerantenne durch die MR-Sendesignale besonders verhindert wird und dennoch mögliche Messzeiten besonders effektiv nutzbar sind. In anderen Worten nutzt das Radiometer die Zeit der Sendephasen, um das Rauschsignal von der als Referenz dienenden Rauschquelle aufzunehmen, während in den Nichtsendephasen das Radiometer auf das Objekt bzw. den Patienten geschaltet wird und dabei das Messsignal von der Radiometerantenne aufnimmt. Unter einer Sendephase wird insbesondere eine Zeitdauer oder ein Zeitfenster verstanden, während dessen MR-TX-Signale ausgesandt werden, während unter einer Nichtsendephase insbesondere eine Zeitdauer oder ein Zeitfenster verstanden wird, während dessen keine MR-TX-Signale ausgesandt werden. Hierbei wird ausgenutzt, dass eine Beeinflussung der Referenz-Rauschsignale von der Rauschquelle während einer Sendephase gering bis vernachlässigbar gering ist.
Nichtsendephasen können beispielsweise Zeitdauern bzw. Phasen zwischen Aussendungen von Anregungspulsen einer MR-Sequenz mit mehreren Anregungspulsen (Spinechozug) sein. Nichtsendephasen können alternativ oder zusätzlich Zeitdauern bzw. Phasen vor oder nach MR-Sequenzen sein, was besonders vorteilhaft ist, weil dann auch keine MR-Antwortsignale des Patienten auftreten.

Es ist eine Ausgestaltung, dass der Dicke-Schalter während Sendephasen des MR-Systems umgeschaltet wird und während Nichtsendephasen des MR-Systems abwechselnd mit der Antenne oder der Rauschquelle verbunden ist. So wird eine Beeinflussung des Referenz-Rauschsignals durch MR-Sendesignale besonders effektiv verhindert.

Es ist eine Weiterbildung, dass das Radiometer nur während Nichtsendephasen des MR-Systems betrieben wird, also z.B. nur während Nichtsendephasen des MR-Systems umgeschaltet wird und nur während Nichtsendephasen des MR-Systems aufgenommene Mess- und Referenzsignale ausgewertet werden. Auch hierdurch wird eine Beeinflussung der Messung des thermischen Rauschens des Patienten durch MR-Signale (zuverlässig verhindert. Zudem ist diese Weiterbildung besonders einfach umsetzbar, da keine Synchronisation einer Umschaltung des Dicke-Schalters während MR-Sendephasen berücksichtigt zu werden braucht.

Es ist eine Ausgestaltung, dass ein Nutzband (d.h., das zur Messung des thermischen Rauschen verwendete Frequenzband) des Radiometers außerhalb eines MR-Bands (d.h., des zur für MR-Signale genutzten Frequenzbands) liegt. Dies ergibt den Vorteil, dass eine Beeinflussung der radiometrischen Temperaturmessung durch MR-Signale besonders gering ist. In anderen Worten liegt, um Störungen der Rauschsignale durch MR-Signale (MR-Sende (TX)- und ggf. auch durch MR-Empfangs(RX)- Signale) zu vermeiden, das Nutzband des Radiometers außerhalb des MR-Frequenzbandes. Dies kann z.B. dadurch umgesetzt sein, dass die Radiometerantenne ein Nutzband aufweist, das außerhalb des MR-Bands liegt. Das MR-Bands umfasst insbesondere eine Präzessionsfrequenz von Protonen und liegt etwa bei 42,5 MHz/T. Bei einem MR-Gerät mit einem B0-Feld der Stärke von 7 Tesla reicht das MR-Band z.B. bis ca. 300 MHz.

Es ist eine zur Erreichung einer hohen Bandbreite vorteilhafte Weiterbildung, dass das Nutzband des Radiometers oberhalb des MR-Bands liegt, also bei höheren Frequenzen. Es ist eine für eine effektive Signaltrennung besonders vorteilhafte Weiterbildung, dass ein Frequenzabstand zwischen dem MR-Band und dem Nutzband des Radiometers mindestens 5 MHz beträgt, insbesondere mindestens 10 MHz. Jedoch sind größere Frequenzabstände noch geeigneter zur Signaltrennung. Besonders geeignet ist ein Frequenzabstand von mindestens 50 MHz, speziell von mindestens 100 MHz. Bei einem 1,5 T-MR-Scanner beträgt somit eine untere Grenze des Nutzbands der Radiometerantenne vorteilhafterweise mindestens ca. 70 MHz, insbesondere mindestens ca. 150 MHz, bei einem 7 T-MR-Scanner mindestens ca. 303 MHz, insbesondere mindestens ca. 400 MHz, usw.

Eine obere Grenze des Nutzbands des Radiometers ist grundsätzlich nicht beschränkt und kann z.B. nicht mehr als 60 GHz betragen, insbesondere nicht mehr als 10 GHz. Dabei kann bei der Auslegung des Nutzbands berücksichtigt werden, dass eine Messtiefe des Radiometers mit steigender Messfrequenz abnimmt, so dass dann, wenn eine Messung der Körpertemperatur besonders tief in dem Patienten gewünscht wird, möglichst geringe Nutzfrequenzen eingestellt werden sollten. Es hat sich ergeben, dass speziell ein Frequenzabstand im Bereich zwischen 50 MHz und 150 MHz einen guten Kompromiss zwischen einer Verhinderung eines Übersprechens und einer ausreichend tief in den Patienten sehenden Temperaturmessung ergibt.

Es ist eine Ausgestaltung, dass das Radiometer einen rauscharmen Verstärker aufweist und zumindest ein durch die Radiometerantenne gemessenes und dann durch den Verstärker verstärktes Messsignal bzw. Rauschsignal einer Auswerteeinrichtung zugeführt wird und mittels der Auswerteeinrichtung ausgewertet wird, z.B. zur Bestimmung der Körpertemperatur und/oder zum Vergleich mit entsprechenden Grenztemperaturen. Beruhend auf der Auswertung kann die Signalstärke des MR-Sendesignals automatisch oder nach Nutzereingabe angepasst werden. Das aufgenommene und verstärkte Rauschsignal kann in analoger oder digitaler Form an die Auswerteeinrichtung übermittelt werden.

Die Auswerteeinrichtung kann eine von der Steuereinrichtung unterschiedliche, aber damit gekoppelte Einrichtung sein. Es ist eine Weiterbildung, dass die Auswerteeinrichtung in die MR-Körperspule integriert ist, wobei dann insbesondere nur noch ein - digitales oder analoges - ausgewertetes Signal von der MR-Körperspule an die dazu externe Steuereinrichtung übermittelt wird. Es ist eine andere Weiterbildung, dass die Auswerteeinrichtung extern von der MR-Körperspule angeordnet ist. Die Auswertung geschieht somit außerhalb der MR-Körperspule, wodurch die MR-Körperspule vorteilhafterweise besonders einfach und preiswert ausgestaltbar ist. Die Auswerteeinrichtung kann in einer Weiterbildung in die Steuereinrichtung integriert sein.

Es ist eine Weiterbildung, dass das der Auswerteeinrichtung zugeführte Rauschsignal seine Originalfrequenz aufweist. Diese Weiterbildung lässt eine besonders einfache und preiswerte MR-Körperspule bereitstellen.

Es ist eine Ausgestaltung, dass das der Auswerteeinrichtung zugeführte Rauschsignal eine konvertierte Frequenz (Trägerfrequenz) aufweist. Das verstärkte Rauschsignal wird in diesem Fall noch in der MR-Körperspule frequenzkonvertiert und in einem anderen Frequenzband nach außen übertragen, insbesondere an die Steuerübertragung übertragen. Insbesondere falls Rauschsignal vor seiner Aussendung abwärtsgewandelt wird, wird der Vorteil erreicht, dass Kabeldämpfungen klein gehalten werden können und evtl. bereits vorhandene Komponenten der MR-Körperspule, die sonst z.B. für eine Signalübertragung von MR-Signalen genutzt werden, mitgenutzt werden können. Die Abwärtswandlung kann mit beliebigen bekannten Methoden durchgeführt werden, z.B. durch Aufmodulation des Messignals auf eine Trägerwelle mit geringerer Frequenz.

Es ist eine Ausgestaltung, dass die gemessene Temperatur oder Temperaturverteilung an einer Nutzerschnittstelle des MR-Systems angezeigt wird, z.B. an einem Nutzerarbeitsplatz. Dies ermöglicht eine Überwachung der Körpertemperatur durch einen Bediener, was eine Sicherheit für einen Patienten weiter erhöhen kann. Insbesondere kann die Temperatur bzw. Temperaturverteilung dazu genutzt werden, um dem Bediener einen Parameter zur Überwachung des Zustandes / Wohlbefindens des Patienten an die Hand zu geben (wie z.B. auch Puls, Atmung oder Sauerstoffsättigung) oder um zu erkennen, wie der (evtl. durch Krankheit geschwächte) Körper auf die SAR-Last der MR-Untersuchung reagiert.

Die Aufgabe wird auch gelöst durch eine MR-Körperspule zur Nutzung in dem oben beschriebenen Verfahren. Die MR-Körperspule kann analog zu dem Verfahren ausgebildet werden und ergibt die gleichen Vorteile.

Die MR-Körperspule weist in einer Weiterbildung außer zur MR-Messung üblichen Komponenten wie mindestens einer MR-Antenne usw. mindestens eine Radiometerantenne und einen Verstärker zur Verstärkung der von der Radiometerantenne empfangenden Messignale auf. Der Verstärker ist insbesondere ein rauscharmer Verstärker. Eine solche MR-Körperspule lässt sich besonders preiswert ausgestalten. Die mindestens eine Radiometerantenne unterscheidet sich vorteilhafterweise von der mindestens einen MR-Antenne.

Es ist eine Ausgestaltung, dass die MR-Körperspule zusätzlich eine Rauschquelle und einen Dicke-Schalter eines Dicke-Radiometers aufweist. Dadurch lassen sich vorteilhafterweise Signalwege der Rauschsignale kurz halten.

Alternativ können die Rauschquelle und der Dicke-Schalter außerhalb der MR-Körperspule angeordnet sein, z.B. in einer Auswerteeinrichtung. Dies ergibt den Vorteil, dass die MR-Körperspule besonders einfach und preiswert ausgestaltbar ist und zudem das von der Rauschquelle erzeugte Referenz-Rauschsignal durch die MR-Signale nicht beeinflusst wird.

Es ist eine Weiterbildung, dass die MR-Körperspule zumindest einen Teil der Auswerteeinrichtung aufweist. Dadurch lassen sich vorteilhafterweise Signalwege der Rauschsignale ganz besonders kurz halten.

Es ist eine Ausgestaltung, dass die MR-Körperspule ein für das MR-Band sperrend ausgelegtes Eingangs-Sperrfilter zwischen der Radiometerantenne und dem Verstärker aufweist. Das Eingangs-Sperrfilter ermöglicht es besonders zuverlässig, dass MR-Signale die von der Radiometerantenne aufgenommenen Messignale nicht beeinflussen, da sie blockiert werden. Auch wird so besonders zuverlässig erreicht, dass das Radiometer während MR-Sendephasen nicht übersteuert. Es ist eine Weiterbildung, dass das Eingangs-Sperrfilter dem Dickeschalter nachgeschaltet ist, da so auch das von der Rauschquelle erzeugte Referenz-Rauschsignal der gleichen Behandlung durch das Eingangs-Sperrfilter unterzogen wird wie das Messsignal und damit auch einen ggf. auftretenden gleichen Leistungsverlust erfährt. So wiederum wird eine Genauigkeit der radiometrischen Temperaturmessung verbessert.

Es ist eine Weiterbildung, dass die Radiometerantenne innerhalb eines Gehäuses der MR-Körperspule angeordnet ist. Es ist eine Weiterbildung, dass das Gehäuse im Bereich zwischen der Radiometerantenne und der vorgesehenen Position des Patienten impedanzangepasst ist. Dadurch können vorteilhafterweise Stehwelleneffekte zwischen Patient und Radiometerantenne vermieden werden. Die Impedanzanpassung kann z.B. durch entsprechende Anpassung einer Dicke des Gehäuses in diesem Bereich und/oder durch Wahl einer Dielektrizitätskonstanten der Gehäusewand in diesem Bereich erreicht werden. Für den Fall, dass die MR-Körperspule dazu vorgesehen ist, unterhalb eines Patienten angeordnet zu sein (z.B. eine Wirbelsäulenspule, eine Kopfspule oder eine Kopf/Nacken-Spule), kann auch ein Kissen, auf welchem der Patient zum Liegen vorgesehen ist, alternativ oder zusätzlich zur Impedanzanpassung ausgelegt sein, beispielsweise durch seine Materialwahl.

Es ist eine Weiterbildung, dass die MR-Körperspule eine Kopfspule, eine Kopf/Nackenspule, eine Wirbelsäulenspule und/oder eine Abdomenspule ist.

Die Aufgabe wird ferner gelöst durch ein MR-System, das zur Durchführung des Verfahrens wie oben beschrieben ausgebildet ist und zumindest eine MR-Körperspule wie oben beschrieben sowie eine mit der mindestens einen MR-Körperspule verbundene Steuereinrichtung aufweist. Das MR-System kann analog zu dem Verfahren ausgebildet werden und ergibt die gleichen Vorteile. Es ist eine Weiterbildung, dass das MR-System ferner einen insbesondere durch die Steuereinrichtung steuerbaren MR-Scanner umfasst.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden schematischen Beschreibung von Ausführungsbeispielen, die im Zusammenhang mit den Zeichnungen näher erläutert werden. Dabei können zur Übersichtlichkeit gleiche oder gleichwirkende Elemente mit gleichen Bezugszeichen versehen sein.

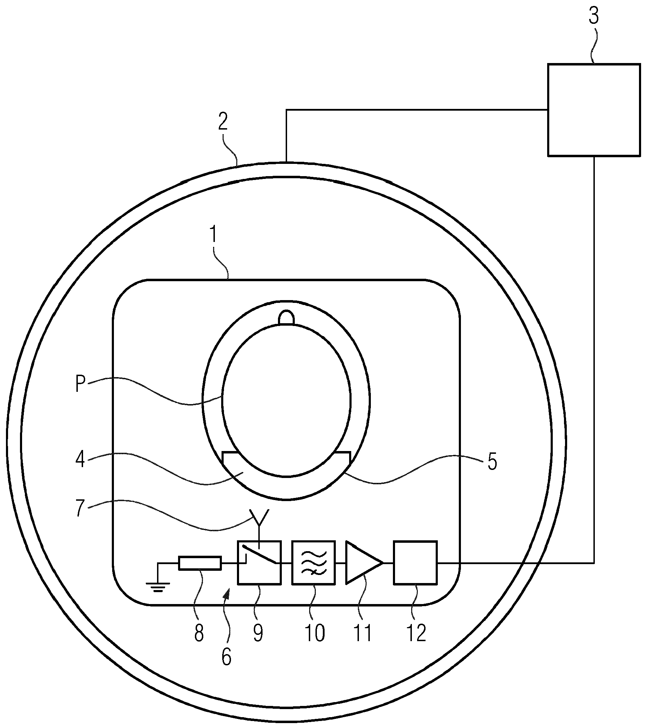

**Fig.1** zeigt als Schnittdarstellung in Ansicht von einer Kopfseite eine Skizze eines MR-Systems mit einer MR-Körperspule 1, einem MR-Scanner 2 und einer mit dem MR-Scanner 2 und der MR-Körperspule 1 verbundenen Steuereinrichtung 3. Der MR-Scanner 2 ist insbesondere ein Hochfeldscanner mit einer Stärke des B0-Felds von mindestens 1,5 T, z.B. von 1,5 T, 3 T, 7 T, 10 T usw. Das MR-Frequenzband des MR-Scanners 2 liegt im Bereich von 42,4 MHz/T. Der MR-Scanner 2 ist wie grundsätzlich bekannt mit MR-Sendeantennen (o. Abb.) ausgerüstet, um während eines MR-Scans MR-Pulse mit Frequenzen im MR-Band auszusenden, z.B. im Rahmen von Echozügen. Die MR-Antwortsignale eines Patienten P werden von MR-Empfangsantennen (o. Abb.) der MR-Körperspule 1 aufgenommen. Die Ansteuerung des MR-Scanners 2, die Auswertung der von den MR-Empfangsantennen aufgenommenen Signale sowie eine Bildgebung werden hier auf grundsätzlich bekannte Weise von der Steuereinrichtung 3 übernommen.

Die MR-Körperspule 1 ist hier beispielhaft als eine Kopfspule zur Untersuchung einer Kopfregion des Patienten P ausgebildet. Der Kopf des Patienten P liegt über ein Kissen 4 auf einer Gehäusewand 5 der MR-Körperspule 1 auf.

In der MR-Körperspule 1 befinden sich zumindest einige Komponenten eines Dicke-Radiometers 6, nämlich hier eine Radiometerantenne 7, eine als geerdeter 50 Ohm-Widerstand ausgebildete Rauschquelle 8, ein mit einer vorgegebenen Umschaltfrequenz zwischen der Radiometerantenne 7 und der Rauschquelle 8 umschaltbarer Dicke-Schalter 9, ein dem Dicke-Schalter 9 nachgeschaltetes Eingangs-Sperrfilter (z.B. ein Tiefpassfilter) 10 zum Blockieren von Frequenzen des MR-Bands, ein dem Eingangs-Sperrfilter 10 nachgeschalteter rauscharmer Verstärker 11 und ggf. weitere elektronische Komponenten 12 wie ein Frequenzwandler zum Herunterwandeln der eingehenden Signale mit z.B. Lokaloszillator, Mischer, IF-Filter, A/D-Wandler, Mikroprozessor, usw.

Die Radiometerantenne 7 und der rauscharme Verstärker 11 arbeiten beide in einem Nutzband, das frequenzbeabstandet oberhalb des MR-Bands liegt. Bei einem 7 T-MR-Scanner beginnt das Nutzband beispielsweise z.B. bei mindestens 303 MHz, vorteilhafterweise bei ca. 400 MHz, und kann z.B. bis 10 GHz oder sogar darüber reichen, z.B. bis 60 GHz. Das Eingangs-Sperrfilter 10 kann entsprechend Frequenzen unterhalb des Nutzbands blockieren.

Mittels eines Frequenzwandlers 12 können die erzeugten Rauschsignale vor Übertragung auf die Steuereinrichtung 3 abwärtsgewandelt werden, um z.B. Kabeldämpfungen klein zu halten und evtl. bereits vorhandene Komponenten der MR-Körperspule 1, die sonst z.B. für eine Signalübertragung von MR-Signalen genutzt werden, mitnutzen zu können.

Die Steuereinrichtung 3 ist dazu eingerichtet, aus den Rauschsignalen des Dicke-Radiometers 6 eine Körpertemperatur des Patienten P im Sichtfeld bzw. Ausleuchtbereich der Radiometerantenne 7 zu bestimmen. Die Körpertemperatur ist oder umfasst vorteilhafterweise eine innere Körpertemperatur des Patienten P, was sich z.B. durch IR-Kameras nicht erreichen lässt.

Die Steuereinrichtung 3 ist auch dazu eingerichtet, z.B. programmiert, die gemessene Körpertemperatur mit einem vorgegebenen Temperaturgrenzwert zu vergleichen und die MR-Sendeleistung des MR-Scanners 2 entsprechend anzupassen.

Um ein Impedanz-Mismatch zwischen dem Patienten P und der Radiometerantenne 7 klein zu halten, sind das Kissen 4 und die Gehäusewand 5 impedanzangepasst ausgebildet.

Die MR-Körperspule 1 kann ein oder mehrere Dicke-Radiometer 6 aufweisen.

Obwohl die Erfindung im Detail durch die gezeigten Ausführungsbeispiele näher illustriert und beschrieben wurde, so ist die Erfindung nicht darauf eingeschränkt und andere Variationen können vom Fachmann hieraus abgeleitet werden, ohne den Schutzumfang der Erfindung zu verlassen.

So können ein oder mehrere der in Fig.1 als zu der MR-Körperspule 1 zugehörig eingezeichnete Komponenten des Dicke-Radiometers 6 alternativ in der Steuereinrichtung 3 vorhanden sein, z.B. die (auch als Rauschdiode bereitstellbare) Rauschquelle 8, der Dicke-Schalter 9, das Eingangs-Sperrfilter 10 und/oder weitere elektronische Komponenten 12.

Allgemein kann unter "ein", "eine" usw. eine Einzahl oder eine Mehrzahl verstanden werden, insbesondere im Sinne von "mindestens ein" oder "ein oder mehrere" usw., solange dies nicht explizit ausgeschlossen ist, z.B. durch den Ausdruck "genau ein" usw.

Auch kann eine Zahlenangabe genau die angegebene Zahl als auch einen üblichen Toleranzbereich umfassen, solange dies nicht explizit ausgeschlossen ist.

## Patentansprüche

1. Verfahren zum Betreiben eines MR-Systems (1, 2, 3) mit mindestens einer MR-Körperspule (1) und einer mit der mindestens einen MR-Körperspule (1) verbundenen Steuereinrichtung (3), bei dem
- mittels mindestens eines Radiometers (6) mindestens eine Körpertemperatur eines durch das jeweilige Radiometer (6) ausleuchtbaren Körperbereichs eines durch das MR-System (1, 2, 3) zu untersuchenden Patienten (P) gemessen wird,
- die gemessene Körpertemperatur mit einer Grenztemperatur verglichen wird und
- eine auf den Patienten eingestrahlte MR-Sendeleistung beruhend auf einem Ergebnis des Vergleichs an die Grenztemperatur angenähert wird.

2. Verfahren nach Anspruch 1, bei dem das Radiometer (6) ein Dicke-Radiometer ist, das eine Radiometerantenne (7), eine Rauschquelle (8) und einen Dicke-Schalter (9) aufweist und der Dicke-Schalter (9) abwechselnd zwischen der Radiometerantenne (7) und der Rauschquelle (8) umgeschaltet wird.

3. Verfahren nach Anspruch 2, bei dem der Dicke-Schalter während Sendephasen des MR-Systems mit der Rauschquelle verbunden wird und während Nichtsendephasen des MR-Systems mit der Radiometerantenne verbunden wird.

4. Verfahren nach Anspruch 2, bei dem der Dicke-Schalter (9) während Sendephasen des MR-Systems (1, 2, 3) umgeschaltet wird, so dass er während Nichtsendephasen des MR-Systems (1, 2, 3) abwechselnd mit der Radiometerantenne (7) und der Rauschquelle (8) verbunden ist.

5. Verfahren nach einem der vorhergehenden Ansprüche, bei dem ein Nutzband des Radiometers (6) außerhalb eines MR-Bands liegt, insbesondere mit einem Frequenzabstand von mindestens 5 MHz oberhalb des MR-Bands liegt.

6. Verfahren nach einem der vorhergehenden Ansprüche, bei dem das Radiometer (6) einen rauscharmen Verstärker (11) aufweist und zumindest ein durch die Radiometerantenne (7) gemessenes und dann durch den Verstärker (11) verstärktes Messsignal einer Auswerteeinrichtung (3), insbesondere der Steuereinrichtung (3), zugeführt wird und mittels der Auswerteeinrichtung (3) ausgewertet wird.

7. Verfahren nach Anspruch 6, bei dem das der Auswerteeinrichtung (3) zugeführte Rauschsignal seine Originalfrequenz aufweist.

8. Verfahren nach Anspruch 6, bei dem das der Auswerteeinrichtung (3) zugeführte Rauschsignal eine abwärtsgewandelte Frequenz aufweist.

9. Verfahren nach einem der vorhergehenden Ansprüche, bei dem die gemessene Körpertemperatur an einer Nutzerschnittstelle des MR-Systems (1, 2, 3) angezeigt wird.

10. Verfahren nach einem der vorhergehenden Ansprüche, bei dem mittels mehrerer Radiometer (6) mit jeweils unterschiedlichen ausleuchtbaren Körperbereichen jeweils mehrere Körpertemperaturen in unterschiedlichen Tiefen des zu untersuchenden Patienten (P) gemessen werden.

11. MR-Körperspule (1), aufweisend mindestens eine Radiometerantenne (7) und einen der Radiometerantenne (7) nachgeschalteten Verstärker (11).

12. MR-Körperspule (1) nach Anspruch 11, ferner aufweisend ein der Radiometerantenne (7) nachgeschaltetes Eingangs-Sperrfilter (10), das für das MR-Band sperrend ausgelegt ist.

13. MR-Körperspule (1) nach einem der Ansprüche 11 bis 12, bei dem eine zwischen der Radiometerantenne (7) und dem Patienten (P) vorgesehene Gehäusewand (5) der MR-Körperspule (1) und/oder ein Kissen (5) zur Auflage des Patienten (P) impedanzangepasst ausgebildet ist.

14. MR-Körperspule (1) nach einem der Ansprüche 11 bis 13, wobei die MR-Körperspule (1) eine Kopfspule, eine Kopf/Nackenspule, eine Wirbelsäulenspule und/oder eine Abdomenspule ist.

15. MR-System (1, 2, 3), aufweisend mindestens einer MR-Körperspule (1) nach einem der Ansprüche 10 bis 13 und einer mit der mindestens einen MR-Körperspule (1) verbundenen Steuereinrichtung (3), wobei das MR-System (1, 2, 3) zur Durchführung des Verfahrens nach einem der Ansprüche 1 bis 10 ausgebildet ist.
